# EUROPÄISCHE PATENTSCHRIFT

(11) **EP 2 392 641 B1**
(45) Veröffentlichungstag und Bekanntmachung des Hinweises auf die Patenterteilung: **15.04.2015**
(21) Anmeldenummer: 11168289.4
(22) Anmeldetag: 31.05.2011
(51) Int. Cl.: C12M 1/107, B01F 5/10, B01F 5/06, C02F 3/30, A01C 3/02, B01F 7/00

(54) **Behälter zur Umwälzung organischer Einsatzstoffe**
Container for circulating organic raw materials
Récipient destiné à faire circuler des matières de départ organiques

(30) Priorität: 01.06.2010 AT 36010 U
(43) Veröffentlichungstag der Anmeldung: 07.12.2011
(73) Patentinhaber: Hörmann Interstall GmbH & Co. KG, 3352 St. Peter/Au (AT)
(72) Erfinder: Thaler, Albert, 4030 Linz (AT); Schweitzer, Franz, 4074 Stroheim (AT); Führer, Franz, 3631 Ottenschlag (AT)
(74) Vertreter: KLIMENT & HENHAPEL

(56) Entgegenhaltungen:
- DE-A1- 3 101 377
- DE-A1- 3 938 248
- US-A- 5 041 217

## Beschreibung

Behälter zur Umwälzung organischer Einsatzstoffe, wie beispielsweise Gülle oder beispielsweise zur Erzeugung von Biogas, umfassend eine Antriebseinheit samt Rotor und Strömungsring/-rohr zur Umwälzung der organischen Einsatzstoffe in einem Ringkanalsystem des Behälters und eine in ihrer vertikalen Position innerhalb des Behälters verstellbare Öffnung in einer einen Querschnitt des Ringkanalsystems ausfüllenden Schottwand, wobei die Schottwand einen sich zumindest über einen Teil der Höhe der Schottwand erstreckenden Durchbruch aufweist und ein in Bezug auf die Tiefe des Behälters oberer Schottvorhang und eine unterer Schottvorhang vorgesehen sind, die einen oberen und einen unteren Abschnitt des Durchbruchs abdecken und zwischen sich die Öffnung ausbilden, wobei die Schottvorhänge als flexible Elemente ausgeführt sind, welche in ihrer Länge jeweils variabel sind und in vertikal verlaufenden Schienenelementen geführt sind, gemäß dem Oberbegriff des Anspruchs 1.

### STAND DER TECHNIK

Solche Behälter zur Umwälzung organischer Einsatzstoffe sind bekannt, beispielsweise aus der Gülletechnik (siehe US-A-504/217). Sinn und Zweck der Umwälzung der organischen Einsatzstoffe ist stets eine gute Durchmischung derselben sowie die Verkleinerung der einzelnen organischen Bestandteile.

Die vorliegende Erfindung bezieht sich auf eine besondere Art solcher Behälter, nämlich mit Zwischenwänden ausgestattete Behälter, welche Zwischenwände so angeordnet sind, dass sie ein vorzugsweise mäanderförmiges Ringkanalsystem ausbilden und zumindest eine einen Strömungsquerschnitt des Ringkanalsystems komplett ausfüllende Schottwand vorgesehen ist.

Die Schottwand wird von einer in ihrer vertikalen Position im Behälter verstellbaren Öffnung durchsetzt, deren Querschnitt geringer als der durchschnittliche Strömungsquerschnitt des Ringkanalsystems ist. Mit dieser Öffnung ist ein Strömungsring/-rohr gekoppelt, in welchem ein Rotor der Antriebseinheit angeordnet ist. Im Betrieb saugt die Antriebseinheit organische Einsatzstoffe in das Strömungsring/-rohr und befördert es strahlartig durch die Öffnung auf die gegenüberliegende Seite der Schottwand.

Zwecks Realisierung der hinsichtlich ihrer vertikalen Position im Behälter variierbaren Öffnung ist die Schottwand mit einem sich zumindest über einen Teil der Höhe der Schottwand erstreckenden Durchbruch versehen, sowie mit einem in Bezug auf die Tiefe des Behälters oberen Schottvorhang und einem unteren Schottvorhang, die einen oberen und einen unteren Abschnitt des Durchbruchs abdecken und zwischen sich die Öffnung ausbilden, wobei die Schottvorhänge als flexible Elemente ausgeführt sind, welche in ihrer Länge jeweils variabel sind und in vertikal verlaufenden Schienenelementen geführt sind. Damit der untere Endbereich des unteren Schottvorhangs nicht aufschwimmt und damit einen Teil des eigentlich abzudeckenden Durchbruchs freigibt, ist der untere Endabschnitt des unteren Schottvorhangs innerhalb des Behälters lagefixiert, beispielsweise durch Verschraubung mit der Schottwand oder dem Behälterboden oder den vertikal verlaufenden Schienelementen, jedenfalls derart, dass der untere Endabschnitt des Durchbruchs vom Schottvorhang komplett abgedeckt wird.

Unter kompletter Abdeckung wird nicht verstanden, dass der Schottvorhang jenen Bereich des Durchbruchs, den er abdeckt, komplett abdichtet. Dies ist schon konstruktionsbedingt nicht möglich, für die Zwecke der Umwälzung der organischen Einsatzstoffe aber auch gar nicht erforderlich. In der Praxis strömt daher ein geringer, für die Einsatzzwecke vernachlässigbarer Prozentsatz der organischen Einsatzstoffe an den Schottvorhängen vorbei durch den Durchbruch.

Die Ankoppelung des Strömungsrings/-rohrs an die Öffnung erfolgt durch Ankoppelung desselben an die untere Kante des oberen Schottvorhangs sowie die obere Kante des unteren Schottvorhangs, beispielsweise über ein rechteckiges Halteelement.

Derart aufgebaute Behälter zur Umwälzung organischer Einsatzstoffe haben den Nachteil, dass aufgrund der oben beschriebenen fixen Montage des unteren Endbereichs des unteren Schottvorhangs zur Wartung des unteren Schottvorhangs eine Entleerung des Behälters oder zumindest eines Absenkung des Füllstandes des Behälters vorgenommen werden muss, um den unteren Endbereich des unteren Schottvorhangs zu lösen und den unteren Schottvorhang abbauen zu können. Aber auch eine Wartung der Antriebseinheit durch Herausheben derselben aus dem Behälter ist ohne Entleerung bzw. Absenkung des Füllstandes nicht möglich, da der untere Schottvorhang mit der Antriebseinheit mittelbar verbunden ist.

Eine damit einhergehende Betriebsunterbrechung verursacht Kosten und kann in verfahrenstechnischer Hinsicht nachteilig sein.

### AUFGABE DER ERFINDUNG

Die Aufgabe der vorliegenden Erfindung ist es, Wartungs- und Reparaturarbeiten an der Schottwand, insbesondere am unteren Schottvorhang und an der Antriebseinheit zu erleichtern und die Betriebseffizienz der Anlage zu steigern.

Eine weitere Aufgabe der vorliegenden Erfindung ist es, den beschriebenen Behälter so zu adaptieren, dass er auch als Fermenter in einem unter Luftabschluss stattfindenden Verfahren zur Erzeugung von Biogas zum Einsatz kommen kann.

### DARSTELLUNG DER ERFINDUNG

Erfindungsgemäß wird dies bei einem gattungsgemäßen Behälter zur Umwälzung organischer Einsatzstoffe dadurch erreicht, indem ein von der Öffnung abgewandter unterer Endbereich des unteren Schottvorhangs mittels einer Haltevorrichtung vorzugsweise in einem unteren Endbereich des Durchbruchs oder in einem unteren Endbereich der Schottwand in einer Betriebsposition lagefixierbar ist, wobei die Haltevorrichtung von einem oberhalb der Öffnung, vorzugsweise von außerhalb des Behälters gelegenen Bereich aus betätigbar und damit die Lagefixierung des unteren Endbereichs des unteren Schottvorhangs aufhebbar ist.

Die Lagefixierung des unteren Endbereichs des unteren Schottvorhangs kann dadurch automatisiert oder aber manuell aufgehoben werden, wodurch der untere Schottvorhang zu Servicearbeiten aus dem Behälter genommen werden kann, ohne diesen entleeren bzw. größtenteils entleeren zu müssen.

Wartungs- und Reparaturarbeiten an der Schottwand und an der Antriebseinheit werden somit wesentlich erleichtert. Eine Betriebsunterbrechung der Anlage während Wartungs- und Reparaturarbeiten ist nicht mehr erforderlich.

In einer bevorzugten Ausführungsvariante der Erfindung umfasst die Haltevorrichtung zwei vom oberen Endbereich zum unteren Endbereich des Durchbruchs der Schottwand oder der Schottwand verlaufende, an seitlichen Endbereichen des Durchbruchs angeordnete Stangenelemente, deren untere Endbereiche am unteren Endbereich des unteren Schottvorhangs angelenkt sind.

Durch die beschriebene bevorzugte Ausbildung der Haltevorrichtung als Stangenelemente, wird zusätzlich zu der oben beschriebenen Lagefixierung bzw. Freigabe der Lagefixierung des unteren Endbereichs des unteren Schottvorhangs auch ermöglicht, den freigegeben unteren Schottvorhang mittels der Stangenelemente aus seiner Betriebsposition in eine Wartungsposition hochzuziehen.

Ein besonders einfaches Freigeben, Lagefixieren und Bewegen des unteren Endbereichs des unteren Schottvorhangs wird gemäß einer bevorzugten Ausführungsvariante der erfindungsgemäßen Stangenelemente dadurch ermöglicht, indem obere Endbereiche der Stangenelemente im oberen Endbereich des Durchbruchs oder der Schottwand, vorzugsweise mittels Schrauben- oder Bolzenelementen an den Schienenelementen fixierbar sind, wobei durch die Fixierung der oberen Endbereiche der Stangenelemente der untere Endbereich des unteren Schottvorhangs in seiner Betriebsposition lagefixiert ist.

In einer bevorzugten Ausführungsvariante der Erfindung sind die Schottvorhänge durch im Wesentlichen horizontal verlaufende, in den Schienenelementen geführte Querstreben in eine Mehrzahl an faltbaren bzw. zusammenschiebbaren Segmenten gegliedert, wobei die Anlenkung der unteren Endbereiche der Haltevorrichtung am unteren Endbereich des unteren Schottvorhangs über eine Querstrebe in diesem unteren Endbereich erfolgt. Auf diese Weise kann der untere Endbereich des unteren Schottvorhangs einfach und zuverlässig in seiner Betriebsposition lagefixiert werden.

Gemäß einer weiteren bevorzugten Ausführungsvariante der Erfindung ist es vorgesehen, dass die Behälterdecke des Behälters oberhalb der Schottwand bzw. der Antriebseinheit einen Service-Deckel aufweist, mit einer in das Innere des Behälters führenden Durchführung, für ein an die Antriebseinheit direkt oder indirekt einhängbares Seil einer Seilwinde, welche an einer über dem Service-Deckel verlaufenden Kranbrücke angeordnet ist.

Auf diese Art und Weise kann während des laufenden Betriebs einerseits die vertikale Adjustierung des Strömungsrings/-rohrs inklusive Antriebseinheit zur Erreichung bestmöglicher Durchmischung des Substratvolumens und zur Vornahme von Servicearbeiten, Antriebseinheit, der obere Schottvorhang sowie der untere Schottvorhang gleichzeitig mittels Seilwinde aus dem Behälter gehoben werden.

Um den Behälter auch als Fermenter in einem unter Luftabschluss stattfindendem Verfahren zum Einsatz bringen zu können, ist es vorgesehen, dass sowohl Service-Deckel als auch Durchführung gasdicht ausgeführt sind.

### KURZE BESCHREIBUNG DER FIGUREN

Die Erfindung wird nun anhand eines Ausführungsbeispiels näher erläutert. Die Zeichnungen sind beispielhaft und sollen den Erfindungsgedanken zwar darlegen, ihn aber keinesfalls einengen oder gar abschließend wiedergeben.

Dabei zeigt:
Fig.1 eine schematische Darstellung eines erfindungsgemäßen Behälters im Grundriss Schnitt AA aus Fig.2)
Fig.2 eine schematische Darstellung eines erfindungsgemäßen Behälters im Seitenriss
Fig.3 eine Ansicht der Schottwand in Draufsicht
Fig.4 die Schottwand aus Fig.3 in Vorderansicht
Fig.5 ein Detail "E" aus Fig.4
Fig.6 ein Detail "F" aus Fig.4
Fig.7 eine Schnittansicht durch die Schottwand gemäß Schnittlinie A-A in Fig.4
Fig.8 ein Detail "B" aus Fig.7
Fig.9 die Schottwand aus Fig.4 in Seitenansicht
Fig.10 ein Detail "C" aus Fig.9
Fig.11 ein Detail "D" aus Fig.9
Fig.12 einen erfindungsgemäßen Behälterdeckel in Vorderansicht
Fig.13 ein Detail "C" aus Fig.12
Fig.14 den Behälterdeckel aus Fig.12 in Draufsicht
Fig.15 eine Seitenansicht des Behälterdeckels aus Fig.14
Fig.16 eine erfindungsgemäße Kranbrücke in Vorderansicht
Fig.17 die Kranbrücke aus Fig.16 in Seitenansicht
Fig.18 die Kranbrücke aus Fig.16 in Draufsicht

### WEGE ZUR AUSFÜHRUNG DER ERFINDUNG

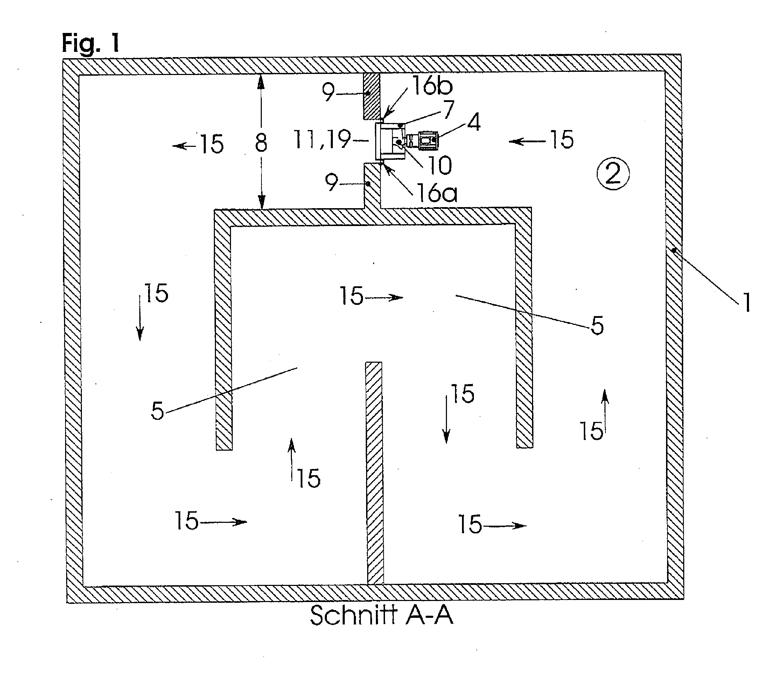

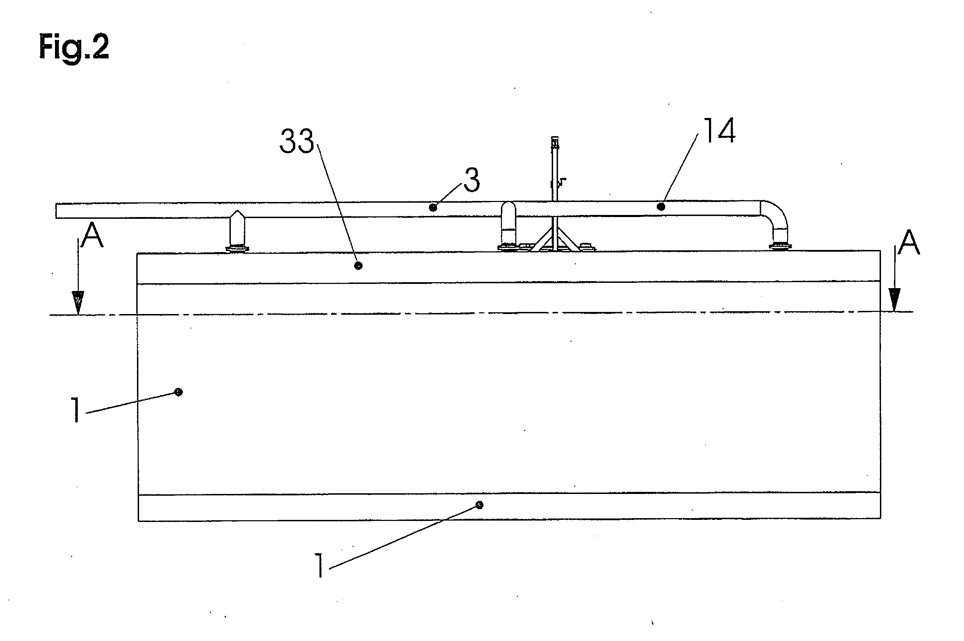

Fig.1 zeigt eine schematische Darstellung eines erfindungsgemäßen Behälters 1 in Schnittdarstellung gemäß Linie AA aus Fig.2.

Im vorliegenden Ausführungsbeispiel ist der Behälter 1 anhand eines Einsatzes zur anaeroben Umwälzung organischer Einsatzstoffe zwecks Herstellung von Biogas beschrieben. Die erfindungsgemäße Haltevorrichtung ist jedoch unabhängig vom Einsatzgebiet und kann daher auch bei anderen gattungsgemäßen Verfahren/Behältern zum Einsatz kommen, in welchen eine Umwälzung organischer Einsatzstoffe erfolgt, beispielsweise bei Behältern zur Umwälzung von Gülle in aerober Atmosphere.

Ein eine Behälterdecke 33 aufweisender Behälter 1 ist in seinem Inneren mit Zwischenwänden versehen, die gemeinsam mit den den Behälter 1 aufbauenden Wänden ein vorzugsweise mäanderförmiges Ringkanalsystem 5 ausbilden. Das Ringkanalsystem 5 weist einen in Bezug auf seine Gesamtlänge errechenbaren mittleren Strömungsquerschnitt auf, der abhängig ist von der Gesamtgeometrie des Ringkanalsystems 5. Diese kann beliebig ausgeführt sein und ist abhängig von diversen Randbedingungen, die an dieser Stelle nicht weiter ausgeführt werden sollen.

In das Ringkanalsystem 5 werden über eine nicht gezeichnete Zuleitung organische Einsatzstoffe 2 eingebracht, zum Zwecke der Gärung/Entgasung über einen bestimmten Zeitraum. Der Behälter 1 weist Mittel 14 (siehe Fig.2) zum Abtransport des durch die Gärung/Entgasung entstehenden Biogases 3 auf.

Um das Entstehen von einer optimalen Entgasung entgegenstehenden Schwimm- und Sinkschichten zu verhindern, ist eine zumindest periodische Umwälzung der Einsatzstoffe im Ringkanalsystem 5 erforderlich. Als besonders vorteilhaft hat sich in diesem Zusammenhang die Umwälzung der Einsatzstoffe durch eine Öffnung erwiesen, deren Umwälzquerschnitt 6 geringer ist als der mittlere Querschnitt des Ringkanalsystems 5.

Dazu ist in einem geeigneten Querschnitt 8 des Ringkanalsystems 5 eine Schottwand 9 angeordnet, die den Querschnitt 8 komplett ausfüllt und das Ringkanalsystem 5 teilt. Die Schottwand 9 ist mit einer den oben erwähnten Umwälzquerschnitt 6 aufweisenden Öffnung 11 versehen, die ein Überströmen der Einsatzstoffe 2 von einem auf der einen Seite der Schottwand 9 befindlichen Abschnitt des Ringkanalsystems 5 in einen Abschnitt auf der anderen Seite der Schottwand 9 ermöglicht.

Um die Überströmung zu bewerkstelligen, ist es erforderlich, die sich im Ringkanalsystem 5 des Gärbehälters 1 befindlichen Einsatzstoffe 2 zumindest periodisch umzuwälzen. Zu diesem Zweck ist eine Antriebseinheit 4 vorgesehen, die einen Rotor 10 aufweist, der in einem Strömungsring/-rohr 7 angeordnet ist.

Der Strömungsring/-rohr 7 ist mehr oder weniger dicht (geringe Leckagen, die ein am Strömungsring/-rohr seitliches Vorbeiströmen durch die Öffnung 11 bewirken, wirken sich nicht störend aus) an die Öffnung 11 angekoppelt, so dass die Antriebseinheit 4 die Einsatzstoffe 2 in Umwälzrichtung 15 strahlförmig durch den Umwälzquerschnitt 6 befördert.

Um das Überströmen der Einsatzstoffe 2 durch den Umwälzquerschnitt 6 in unterschiedlichen vertikalen Positionen im Gärbehälter 1 zu ermöglichen, ist es erfindungsgemäß vorgesehen, dass die den Umwälzquerschnitt 6 aufweisende Öffnung 11 entlang der Schottwand 9 verschiebbar angeordnet ist. Der Begriff vertikal ist in diesem Zusammenhang auf die Betriebsposition des Gärbehälters abgestellt.

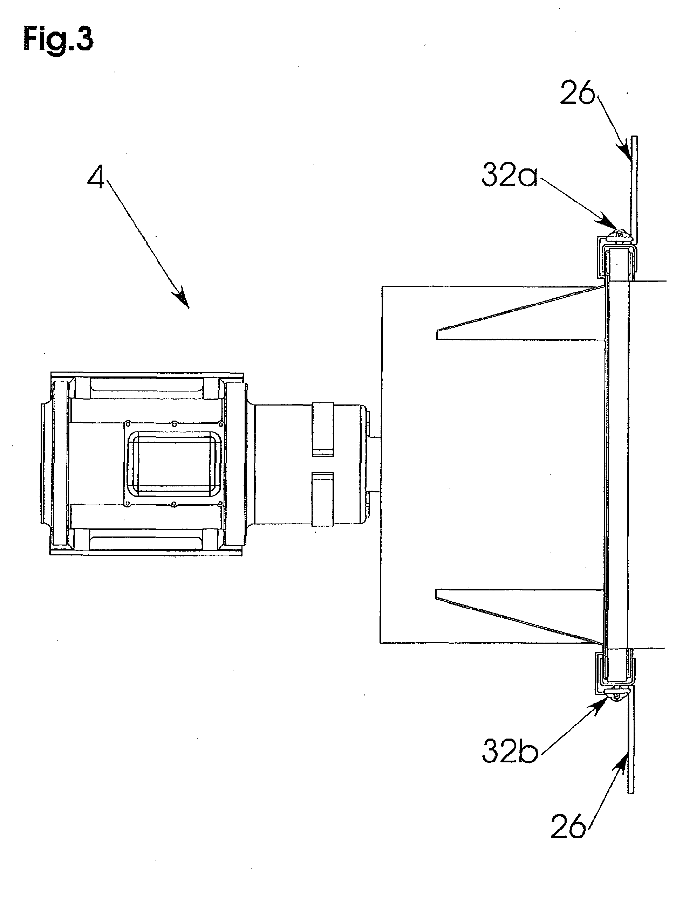

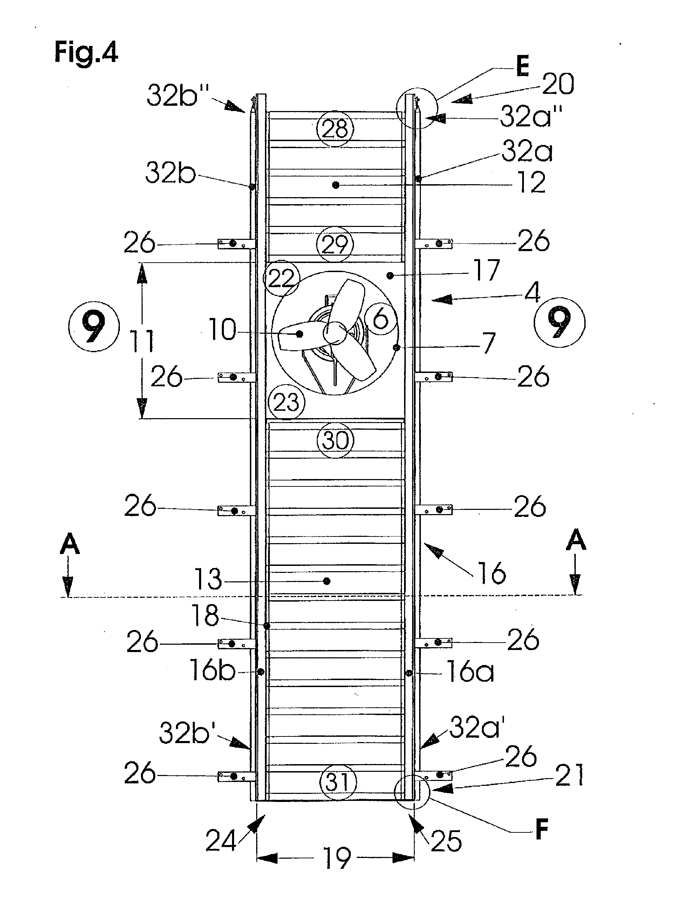

Dazu weist die Schottwand 9 einen sich über die gesamte bzw. einen Großteil der Höhe der Schottwand 9 (siehe Fig.3,4 und 7,9) verlaufenden Durchbruch 19 auf. An einer Seite der Schottwand 9, im Bereich der Kanten des Durchbruchs 19, ist ein Schienensystem 16, beispielsweise über Montagelaschen 26, angeordnet, über welches Schienensystem 16 die Ankoppelung des Strömungsrings/-rohres 7 an den Durchbruch 19 mittels einer Halterung 17 erfolgt.

Die Halterung 17 ist im Schienensystem 16 verschiebbar. Die verbleibenden Bereiche des Durchbruchs 19 oberhalb und unterhalb der Halterung 17 sind durch jalousie- oder vorhangartige, in ihrer Länge variable, beispielsweise zusammenschiebbare Elemente abgedeckt, so dass als Öffnung 11 in der Schottwand 9 jener Bereich zwischen unterster Kante des oberen jalousie- oder vorhangartigen Elementes und der oberen Kante des unteren jalousie- oder vorhangartigen Elementes fungiert.

In diesem Bereich ist der Strömungsring/-rohr 7 samt Halterung 17 an den Durchbruch 19 verschiebbar gekoppelt.

Auf diese Art und Weise kann der Strömungsring/-rohr 7 inkl. Halterung 17 und Antriebseinheit 4 entlang des Schienensystems 16 d.h. entlang des Durchbruchs 19 verfahren werden, wodurch sich eine entlang der Schottwand 9 verschiebbare Öffnung 11 ergibt.

Je nach Ausführung der Halterung 17 kann diese auch den Umwälzquerschnitt 6 definieren, indem sie einen Teil der Öffnung abdeckt.

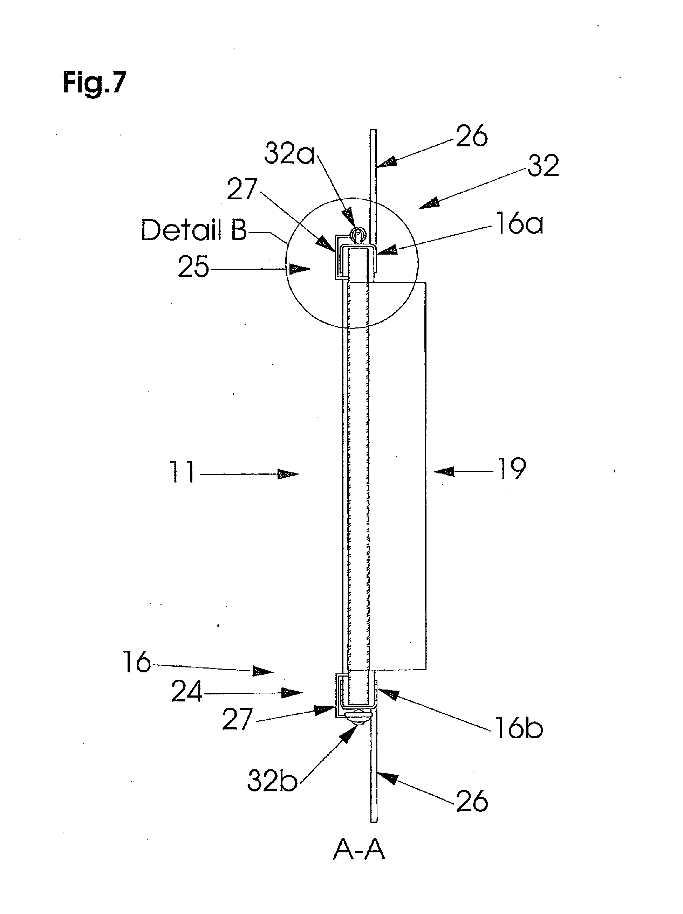

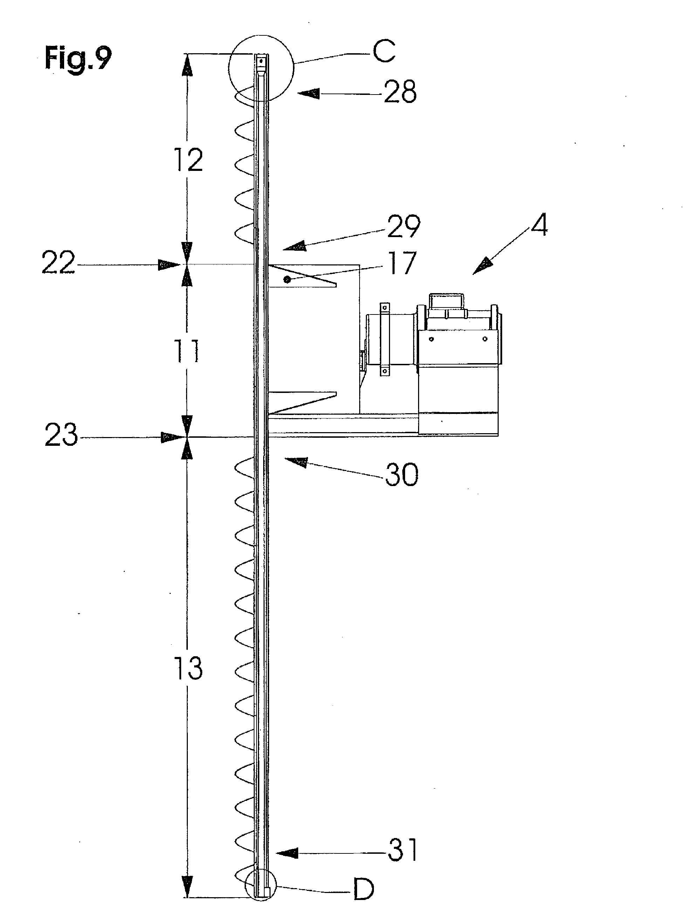

Als jalousie- oder vorhangartige, in ihrer Länge variable Elemente kommen flexible Schottvorhänge 12, 13 zum Einsatz, die aus gewebeverstärktem PVC-Planenmaterial gefertigt sind. Ein Schottvorhang 12 ist mit seinem unteren Endbereich 29 am oberen Ende der Halterung 17 sowie mit seinem oberen Endbereich 28 am oberen Ende des Durchbruchs 19 befestigt, der andere Schottvorhang 13 mit seinem oberen Endbereich 30 am unteren Ende der Halterung 17 (Fig.7,9). Über die Länge der Schottvorhänge 12,13 verteilt sind mit diesen verbundene und im Schienensystem 16 geführte Querstreben 18 angeordnet, wodurch die Schottvorhänge 12,13 eine Mehrzahl an faltbaren bzw. zusammenschiebbaren Segmenten ausbilden.

Erfindungsgemäß ist es vorgesehen, dass der Öffnung 11 abgewandter unterer Endbereich 31 des unteren Schottvorhangs 13 mittels einer Haltevorrichtung 32 vorzugsweise in einem unteren Endbereich 21 des Durchbruchs 19 oder in einem unteren Endbereich der Schottwand in einer Betriebsposition lagefixierbar ist, wobei die Haltevorrichtung 32 von einem oberhalb der Öffnung 11, vorzugsweise von außerhalb des Behälters 1 gelegenen Bereich aus betätigbar und damit die Lagefixierung des unteren Endbereichs 31 des unteren Schottvorhangs 13 aufhebbar ist.

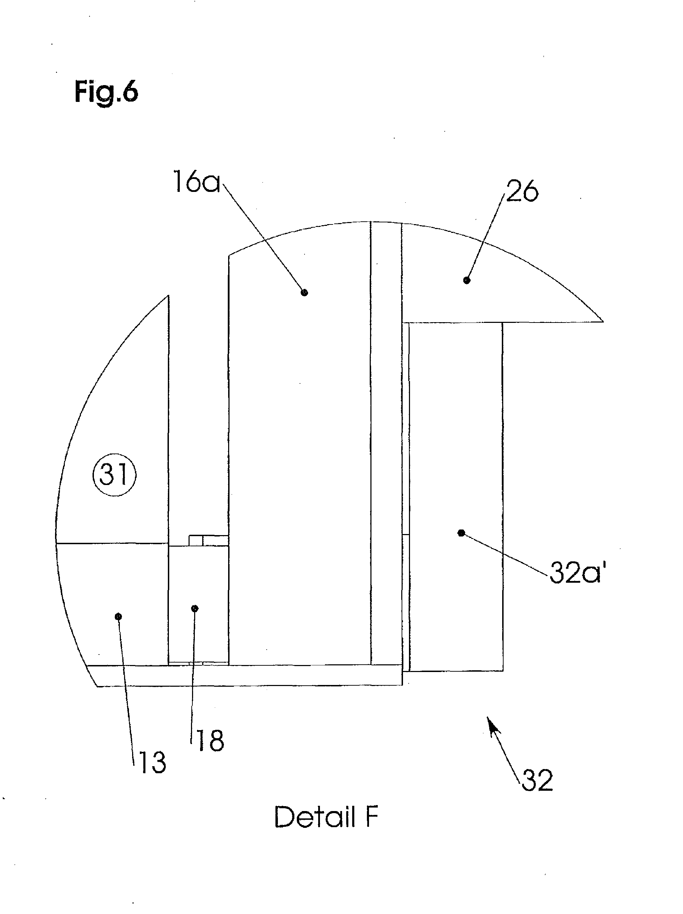

Die in Fig.6 im Detail dargestellte Haltevorrichtung 32 ist somit aus einer den unteren Endbereich 31 des unteren Schottvorhangs 13 lagefixierenden Stellung in eine den unteren Endbereich 31 des unteren Schottvorhangs 13 freigebende Stellung beförderbar.

Die Betätigung der Haltevorrichtung 32 bzw. das Befördern aus der lagefixierenden in die freigebende Stellung kann manuell, z.B. mittels einfachen Aushängens der Haltevorrichtung 32 aus einer korrespondierenden Einhängevorrichtung oder automatisiert, z.B. mittels eines elektrischen Stellantriebs oder einer hydraulischen oder pneumatischen Verstelleinrichtung erfolgen.

Im vorliegenden Ausführungsbeispiel umfasst die Haltevorrichtung 32 zwei vom oberen Endbereich 20 zum unteren Endbereich 21 des Durchbruchs 19 der Schottwand 9 verlaufende, an seitlichen Endbereichen 24, 25 des Durchbruchs 19 angeordnete Stangenelemente 32a, 32b, deren untere Endbereiche 32a', 32b' mit dem unteren Endbereich 31 des unteren Schottvorhangs 13 gekoppelt sind und daher lagefixieren und gegen ein Aufschwimmen sichern.

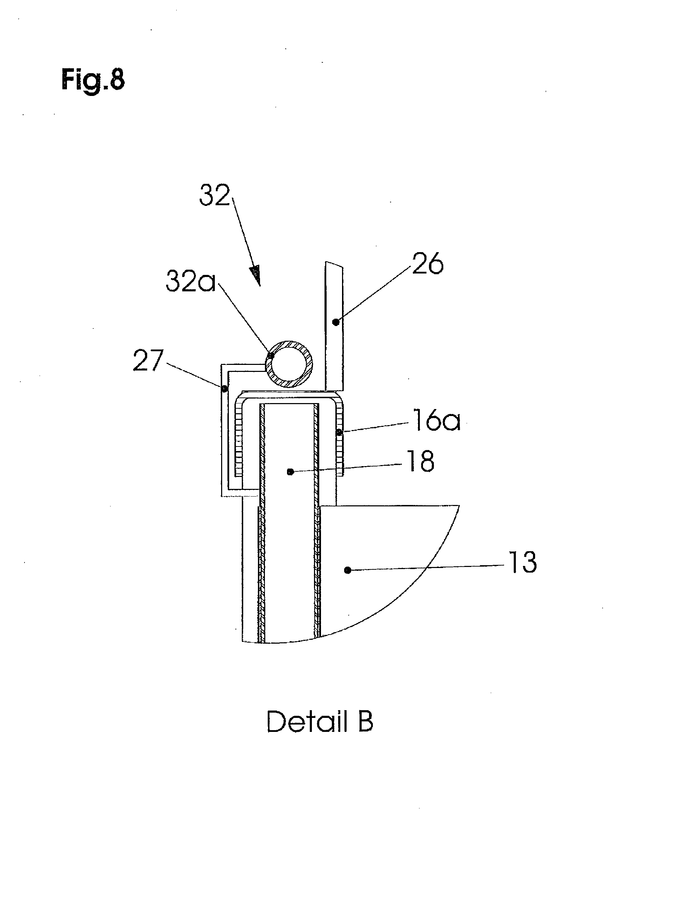

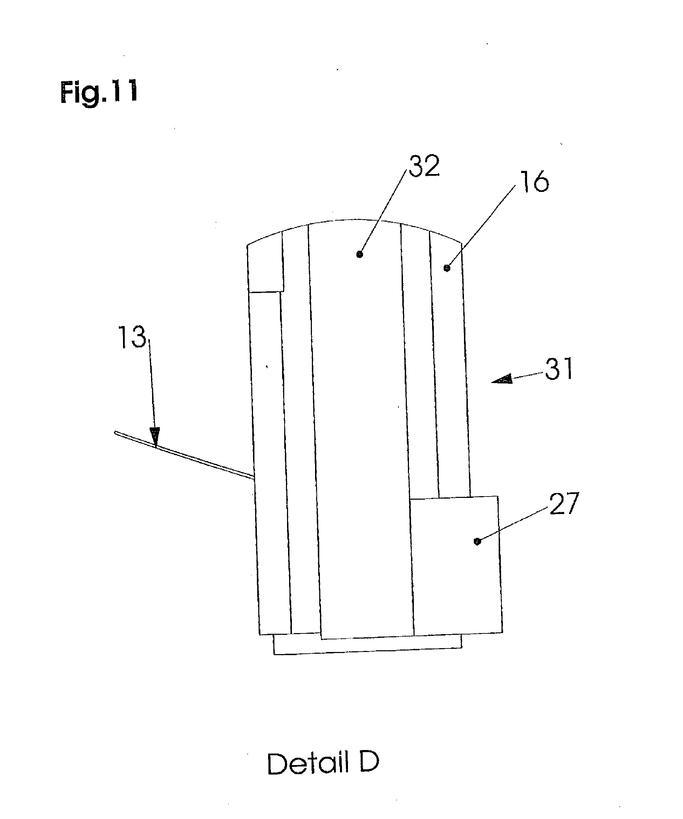

Die unteren Endbereiche 32a', 32b' der Stangenelemente 32a, 32b können z.B. mit in Fig.8 und Fig.11 im Detail ersichtlichen, laschenförmigen Halteelementen 27 versehen sein, welche am unteren Schottvorhangs 13 angelenkt, z.B. angeschraubt oder verschweisst sind.

In einer bevorzugten Ausführungsvariante sind die Stangenelemente 32a, 32b bzw. die Halteelemente 27 an einer im unteren Endbereich 31 des unteren Schottvorhangs 13 angeordneten Querstrebe 18 befestigt.

Der untere Endbereich 31 des unteren Schottvorhangs 13 kann somit im Bedarfsfalle über die unterste Querstrebe 18 mittels der Stangenelemente 32a, 32b entlang der Schienenelemente 16a,16b aus seiner Betriebsposition in eine Wartungsposition hochgezogen werden.

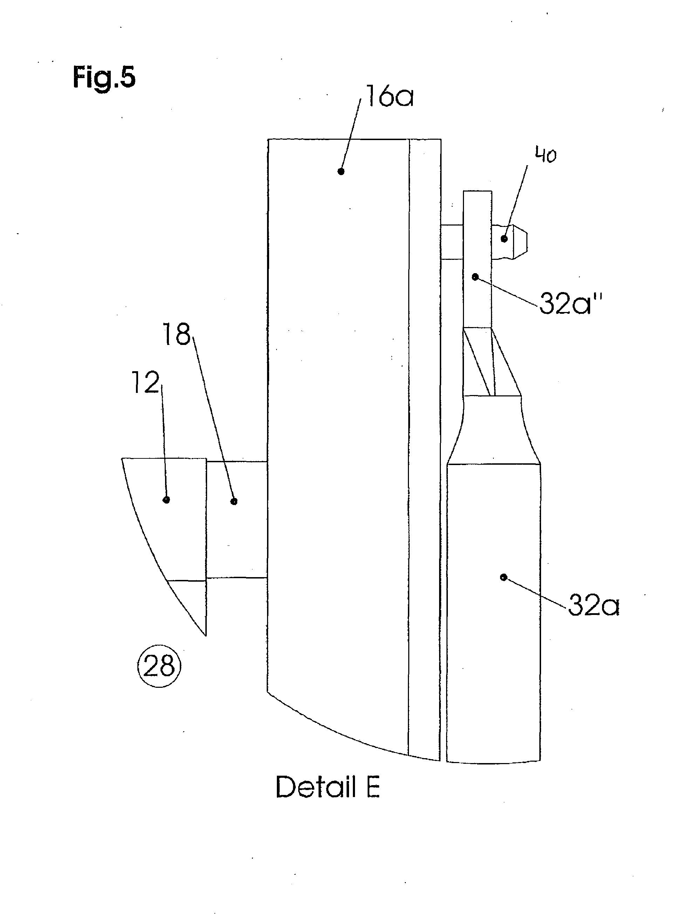

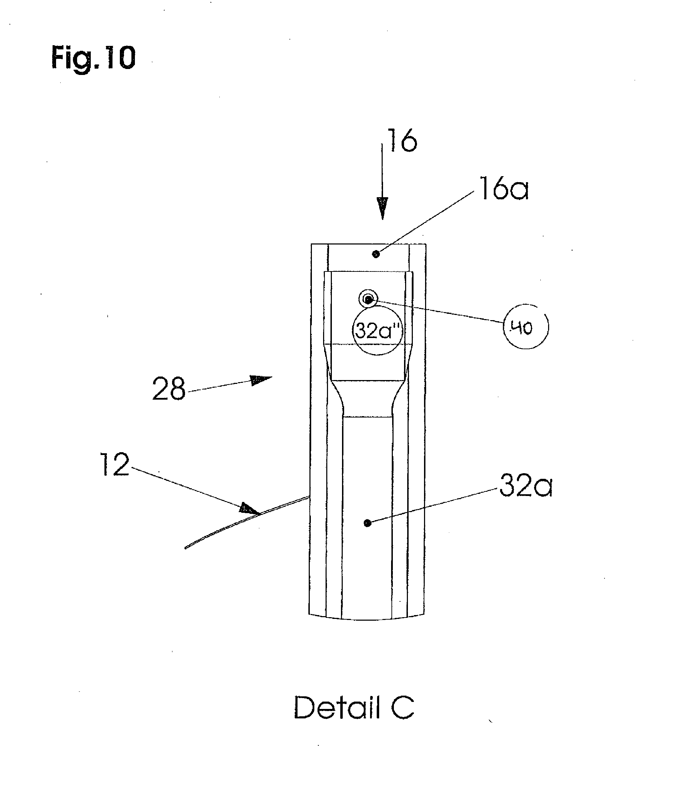

Wie in den Figuren 5 und 10 ersichtlich, sind im vorliegenden Ausführungsbeispiel obere Endbereiche 32a" und 32b" der Stangenelemente 32a, 32b flachgepresst und jeweils mittels eines Schrauben- oder Bolzenelementes 40 an den Schienenelementen 16a, 16b lösbar befestigt. Eine Befestigung der oberen Endbereiche 32a", 32b" der Stangenelemente 32a, 32b erfolgt in einer Position bzw. auf einer Höhe der Schienenelemente 16a, 16b, in welcher der untere Endbereich 31 des unteren Schottvorhangs 13 in seiner Betriebsposition lagefixiert ist.

Selbstverständlich kann die Befestigung der oberen Endbereiche 32a" und 32b" auch an der Schottwand 9 oder an der Behälterdecke 33 erfolgen, ohne vom grundsätzlichen Erfindungsgedanken abzuweichen.

Unabhängig von ihrer konstruktiven Ausführung ist es wesentlich, dass die Haltevorrichtung 32 in der Lage ist, den unteren Endbereich 31 des unteren Schottvorhangs 13, im vorliegenden Fall die unterste oder eine der unteren Querstreben 18 gegen ein vertikal nach oben gerichtetes Verschieben, insbesondere gegen eine Aufschwimmen zu blockieren bzw. freizugeben.

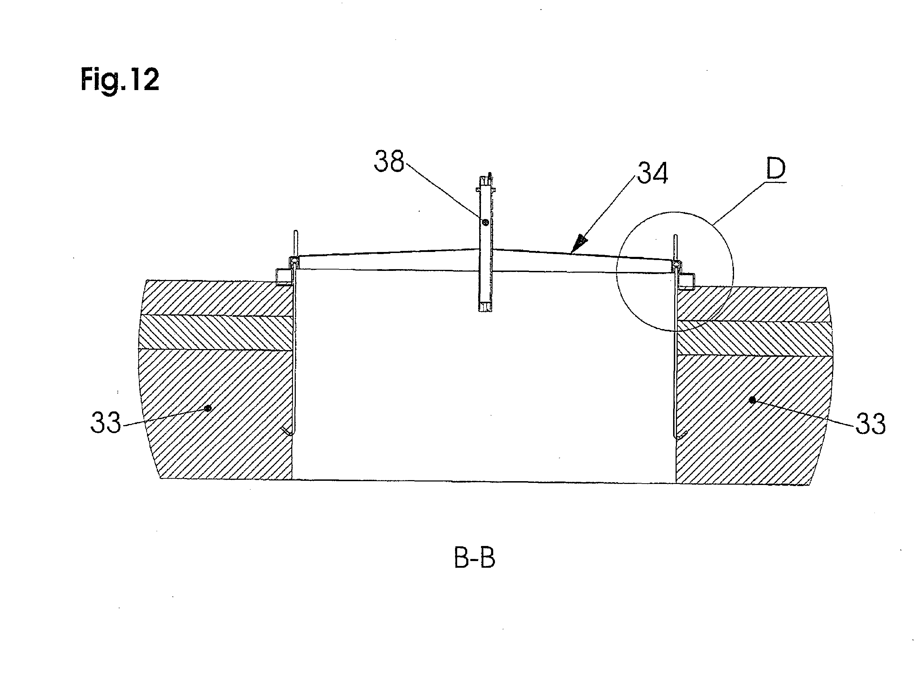

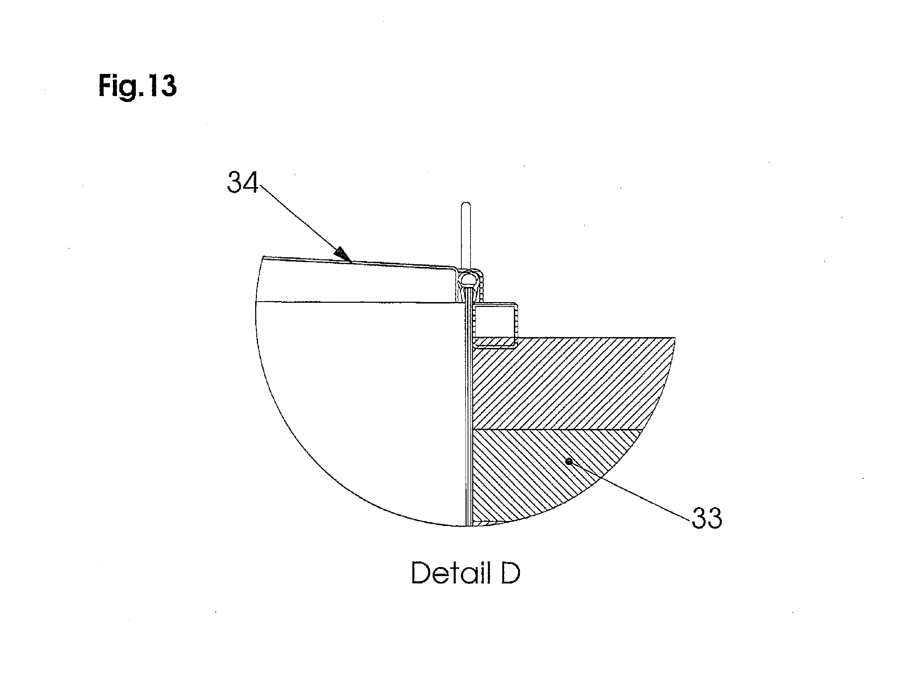

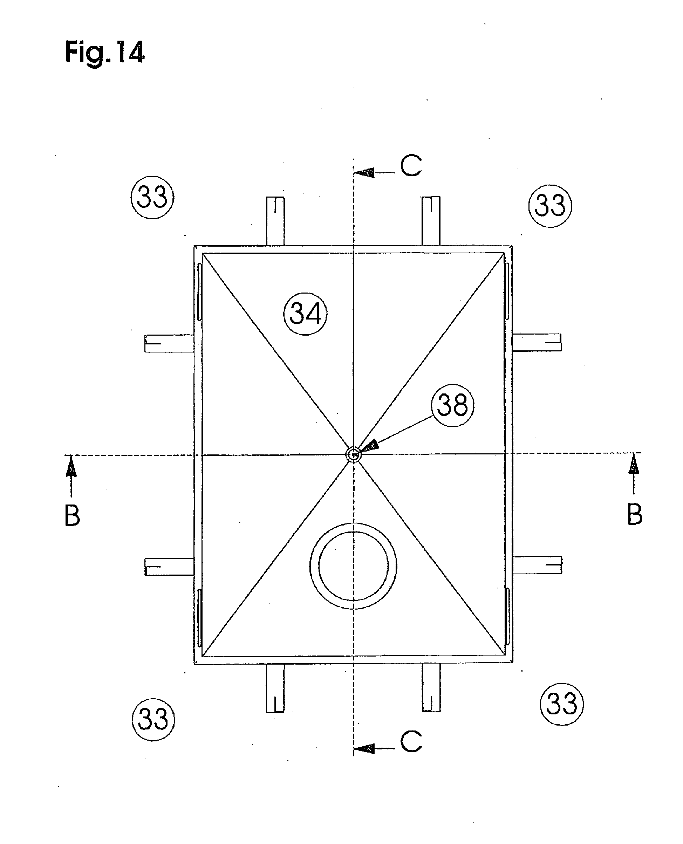

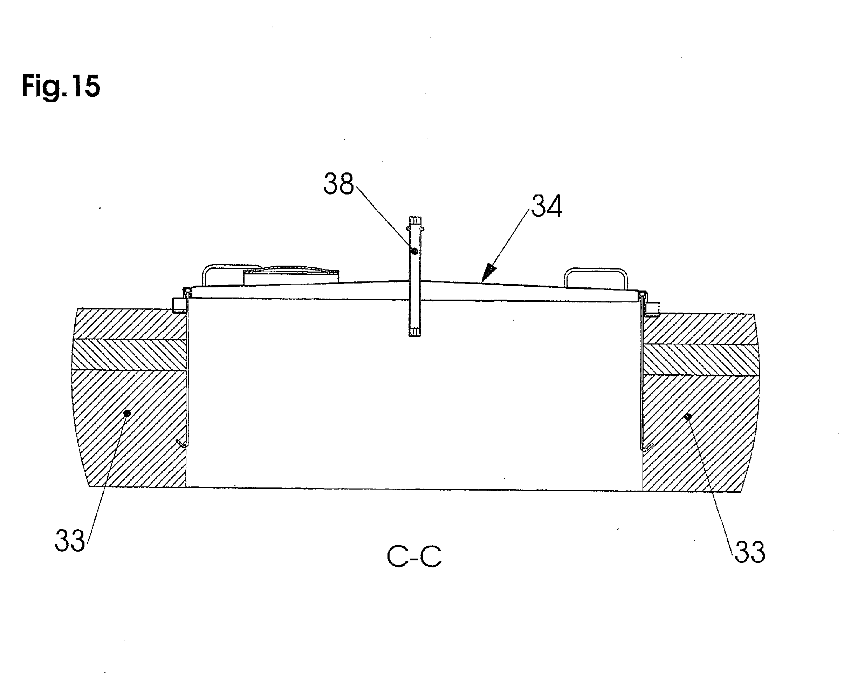

Da die Wartung des unteren Schottvorhangs 13 in der Regel gemeinsam mit der Wartung der Antriebseinheit 4 und des oberen Schottvorhanges 12 erfolgt, ist es vorgesehen, dass die Behälterdecke 33 des Behälters 1 oberhalb der Schottwand 9 bzw. der Antriebseinheit 4 einen Service-Deckel 34 (siehe Figuren 12-15) aufweist, mit einer in das Innere des Behälters 1 führenden Durchführung 38, für ein an die Antriebseinheit 4 direkt oder indirekt einhängbares Seil 39 einer über eine Kurbel 37 betätigbare Seilwinde 35, welche an einer über dem Service-Deckel verlaufenden Kranbrücke 36 angeordnet ist.

Dadurch können mit Hilfe der Haltevorrichtung 32 zunächst der der untere Endbereich 31 des unteren Schottvorhangs 13 aus seiner Lagefixierung gelöst werden und danach die Antriebseinheit 4 samt den beiden an der Antriebseinheit 4 über das Halteelement 17 und den Strömungsring/-rohr 7 samt Rotor 10 verbundenen Schottvorhängen 12,13 nach oben aus dem Behälter 1 gezogen werden.

Um Gasverluste bei Wartungs- und Reparaturarbeiten zu minimieren, ist es vorgesehen, dass eine temporäre Abdeckung (hier nicht näher beschrieben) der Öffnung in der Behälterdecke 33 zum Einsatz kommt.

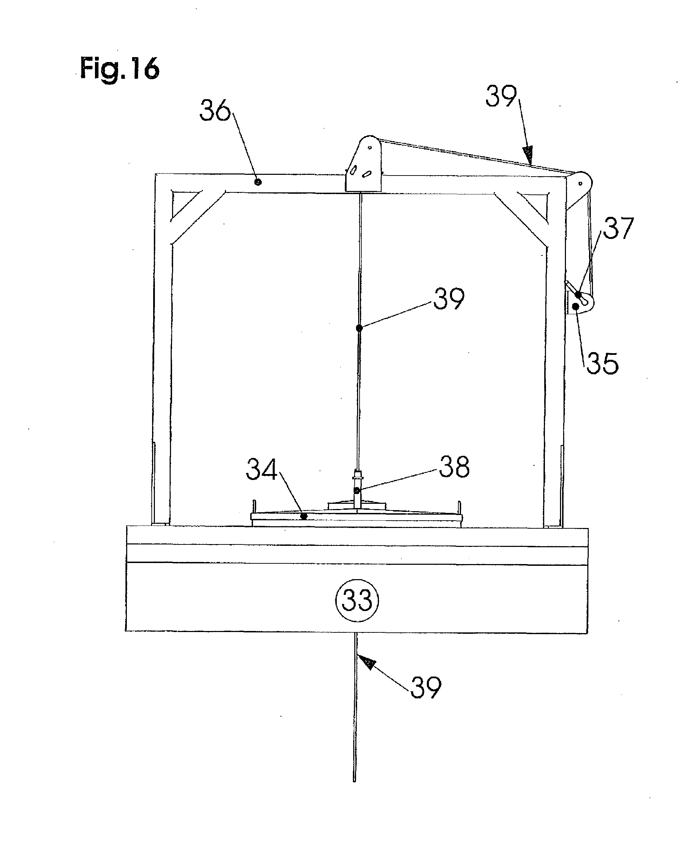

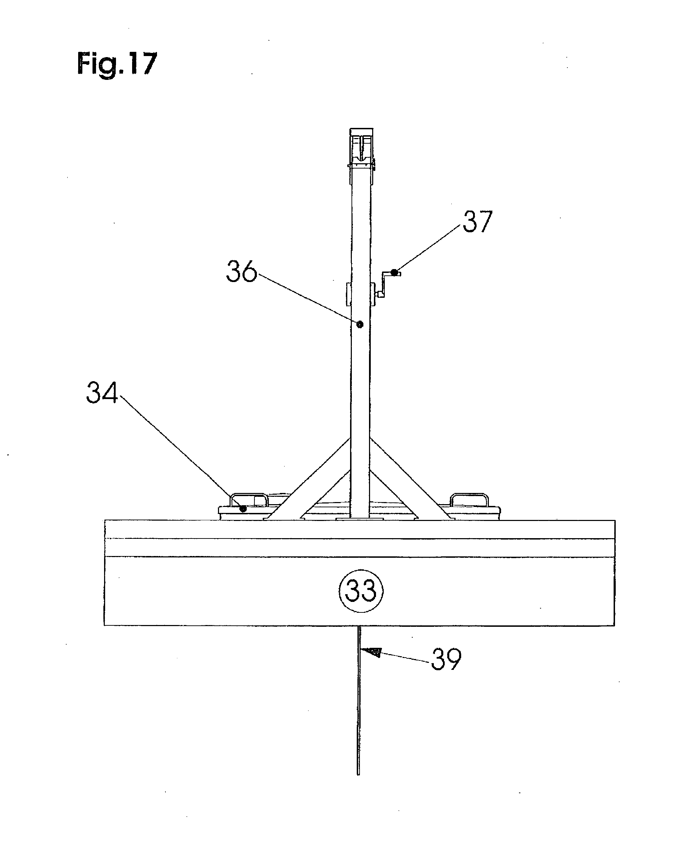

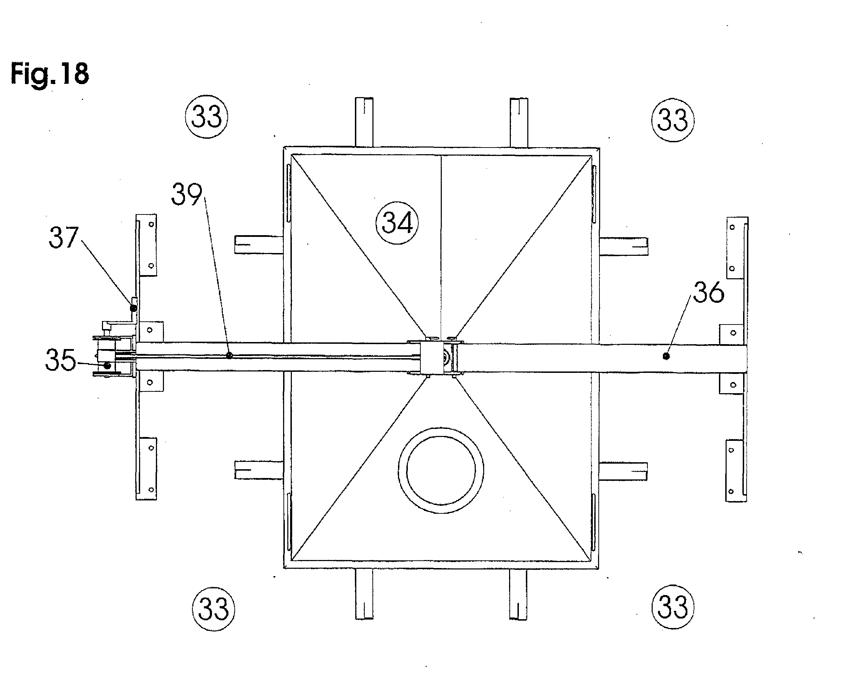

Gemäß einer in den Figuren 16-18 dargestellten Ausführungsvariante der Erfindung ist es vorgesehen, dass auf einer Behälterdecke 33 des Behälters 1 eine den Service-Deckel 34 überspannende Kranbrücke 36 angebracht ist, mittels welcher das Seil 39 der Seilwinde 35 auf- und abwickelbar und somit die Antriebseinheit 4 samt der Öffnung 9 heb- oder senkbar ist.

### BEZUGSZEICHENLISTE

1 Behälter
2 organische Einsatzstoffe
3 Biogas
4 Antriebseinheit
5 Ringkanalsystem
6 Umwälzquerschnitt
7 Strömungsring/-rohr
8 ein Querschnitt des Ringkanalsystems
9 Schottwand
10 Rotor der Antriebseinheit
11 Öffnung in der Schottwand
12 oberer Schottvorhang
13 unterer Schottvorhang
14 Mittel zur Entnahme des Biogases
15 Umwälzrichtung der Einsatzstoffe
16 Schienensystem
16a erstes Schienenelement
16b zweites Schienenelement
17 Halterung
18 Querstreben
19 Durchbruch in der Schottwand
20 oberer Endbereich des Durchbruchs 19
21 unterer Endbereich des Durchbruchs 19
22 oberer Endbereich der Öffnung 11
23 unterer Endbereich der Öffnung 11
24 seitlicher Endbereich des Durchbruchs 19
25 seitlicher Endbereich des Durchbruchs 19
26 Montagelasche
27 Halteelement
28 oberer Endbereich des oberen Schottvorhangs 12
29 unterer Endbereich des oberen Schottvorhangs 12
30 oberer Endbereich des unteren Schottvorhangs 13
31 unterer Endbereich des unteren Schottvorhangs 13
32 Haltevorrichtung
32a erstes Stangenelement
32b zweites Stangenelement
32a' unterer Endbereich des Stangenelementes 32a
32b' unterer Endbereich des Stangenelementes 32b
32a" oberer Endbereich des Stangenelementes 32a
32b" oberer Endbereich des Stangenelementes 32b
33 Behälterdecke
34 Service-Deckel
35 Seilwinde
36 Kranbrücke
37 Kurbel
38 gasdichte Durchführung
39 Seilwindenseil
40 Bolzenelement

## Patentansprüche

1. Behälter (1) zur Umwälzung organischer Einsatzstoffe (2), wie beispielsweise Gülle oder beispielsweise zur Erzeugung von Biogas, umfassend eine Antriebseinheit (4) samt Rotor (10) und Strömungsring/-rohr (7) zur Umwälzung der organischen Einsatzstoffe (2) in einem Ringkanalsystem (5) des Behälters (1) und durch eine in ihrer vertikalen Position innerhalb des Behälters verstellbare Öffnung (11) einer einen Querschnitt (8) des Ringkanalsystems (5) ausfüllenden Schottwand (9), wobei die Schottwand (9) einen sich zumindest über einen Teil der Höhe der Schottwand (9) erstreckenden Durchbruch (19) aufweist und ein in Bezug auf die Tiefe des Behälters (1) oberer Schottvorhang (12) und eine unterer Schottvorhang (13) vorgesehen sind, die einen oberen und einen unteren Abschnitt des Durchbruchs (19) abdecken und zwischen sich die Öffnung (11) ausbilden, wobei die Schottvorhänge (12,13) als flexible Elemente ausgeführt sind, welche in ihrer Länge jeweils variabel sind und in vertikal verlaufenden Schienenelementen (16a,16b) geführt sind, **dadurch gekennzeichnet, dass** ein von der Öffnung (11) abgewandter unterer Endbereich (31) des unteren Schottvorhangs (13) mittels einer Haltevorrichtung (32) vorzugsweise in einem unteren Endbereich (21) des Durchbruchs (19) oder in einem unteren Endbereich der Schottwand in einer Betriebsposition lagefixierbar ist, wobei die Haltevorrichtung (32) von einem oberhalb der Öffnung (11), vorzugsweise von außerhalb des Behälters (1) gelegenen Bereich aus betätigbar und damit die Lagefixierung des unteren Endbereichs (31) des unteren Schottvorhangs (13) aufhebbar ist.

2. Behälter (1) zur Umwälzung organischer Einsatzstoffe (2) nach Anspruch 1, **dadurch gekennzeichnet, dass** die Haltevorrichtung (32) zwei vom oberen Endbereich (20) zum unteren Endbereich (21) des Durchbruchs (19) der Schottwand (9) oder der Schottwand (9) verlaufende, an seitlichen Endbereichen (24, 25) des Durchbruchs (19) angeordnete Stangenelemente (32a, 32b) umfasst, deren untere Endbereiche (32a', 32b') am unteren Endbereich (31) des unteren Schottvorhangs (13) angelenkt sind.

3. Behälter (1) zur Umwälzung organischer Einsatzstoffe (2) nach Anspruch 2, **dadurch gekennzeichnet, dass** obere Endbereiche (32a" und 32b") der Stangenelemente (32a, 32b) im oberen Endbereich (20) des Durchbruchs (19) oder der Schottwand (9), fixierbar sind, wobei durch die Fixierung der oberen Endbereiche (32a", 32b") der Stangenelemente (32a, 32b) der untere Endbereich (31) des unteren Schottvorhangs (13) in seiner Betriebsposition lagefixiert ist.

4. Behälter (1) zur Umwälzung organischer Einsatzstoffe (2) nach Anspruch 2 oder 3, **dadurch gekennzeichnet, dass** die Schottvorhänge (12,13) durch im Wesentlichen horizontal verlaufende, in den Schienenelementen (16a, 16b) geführte Querstreben (18) in eine Mehrzahl an faltbaren bzw. zusammenschiebbaren Segmenten gegliedert sind, wobei die Anlenkung der unteren Endbereiche (32a', 32b') der Haltevorrichtung (32) am unteren Endbereich des unteren Schottvorhangs (13) über eine Querstrebe (18) in diesem unteren Endbereich (31) erfolgt.

5. Behälter (1) zur Umwälzung organischer Einsatzstoffe (2) nach einem der Ansprüche 1 bis 4, **dadurch gekennzeichnet, dass** die Behälterdecke (33) des Behälters (1) oberhalb der Schottwand (9) bzw. der Antriebseinheit (4) einen Service-Deckel (34) aufweist, mit einer in das Innere des Behälters (1) führenden Durchführung (38), für ein an die Antriebseinheit (4) direkt oder indirekt einhängbares Seil (39) einer Seilwinde (35), welche an einer über dem Service-Deckel verlaufenden Kranbrücke (36) angeordnet ist.

6. Behälter (1) zur Umwälzung organischer Einsatzstoffe (2) nach Anspruch 5, **dadurch gekennzeichnet, dass** sowohl Service-Deckel (34) als auch Durchführung (38) gasdicht ausgeführt sind.

## Claims

1. A container (1) for circulating organic raw materials (2) such as slurry or for producing biogas for example, comprising a drive unit (4) together with a rotor (10), and a flow ring/tube (7) for circulating the organic raw materials (2) in a ring channel system (5) of the container (1) and through an opening (11), which is adjustable in its vertical position within the container, of a bulkhead wall (9) filling a cross-section (8) of the ring channel system (5), wherein the bulkhead wall (9) comprises a breakthrough (19) extending over a part of the height of the bulkhead wall (9), and a bulkhead curtain (12), which is an upper curtain with respect to the depth of the container (1), and a bottom bulkhead curtain (13) are provided, which cover an upper and a bottom section of the breakthrough (19) and form the opening (11) between themselves, wherein the bulkhead curtains (12, 13) are arranged as flexible elements which are respectively variable in their length and are guided in vertically extending rail elements (16a, 16b), **characterized in that** a lower end region (31) of the bottom bulkhead curtain (13) facing away from the opening (11) is fixed in position by means of a retaining apparatus (32) preferably in a bottom end region (21) of the breakthrough (19) or in a bottom end region of the bulkhead in an operating position, wherein the retaining apparatus (32) can be actuated from a region situated above the opening (11), preferably from outside of the container (1), and the positional fixing of the bottom end region (31) of the bottom bulkhead curtain (13) can thus be removed.

2. A container (1) for circulating organic raw materials (2) according to claim 1, **characterized in that** the retaining apparatus (32) comprises two rod elements (32a, 32b), which extend from the upper end region (20) to the bottom end region (21) of the breakthrough (19) of the bulkhead wall (9) or of the bulkhead wall (9), and are arranged on the lateral end regions (24, 25) of the breakthrough (19), and whose bottom end regions (32a', 32b') are articulately jointed to the bottom end region (31) of the bottom bulkhead curtain (13).

3. A container (1) for circulating organic raw materials (2) according to claim 2, **characterized in that** the upper end regions (32a", 32b") of the rod elements (32a, 32b) can be fixed in the upper end region (20) of the breakthrough (19) or the bulkhead wall (9), wherein the bottom end region (31) of the bottom bulkhead curtain (13) can be positionally fixed with respect to its operating position by the fixing of the upper end regions (32a", 32b") of the rod elements (32a, 32b).

4. A container (1) for circulating organic raw materials (2) according to claim 2 or 3, **characterized in that** the bulkhead curtains (12, 13) are broken down into a plurality of foldable or collapsible segments by cross members (18) which extend substantially horizontally and are guided in the rail elements (16a, 16b), wherein the linkage of the bottom end regions (32a', 32b') of the retaining apparatus (32) to the bottom end region of the bottom bulkhead curtain (13) occurs by a cross member (18) in said bottom end region (31).

5. A container (1) for circulating organic raw materials (2) according to one of the claims 1 to 4, **characterized in that** the container cover (33) of the container (1) comprises a service cover (34) above the bulkhead wall (9) or the drive unit (4), comprising a passage (38) leading into the interior of the container (1), for a cable (39) of a cable winch (35), which cable can be hooked directly or indirectly into the drive unit (4), which winch is arranged on a crane bridge (36) extending above the service cover.

6. A container (1) for circulating organic raw materials (2) according to claim 5, **characterized in that** both the service cover (34) and also the passage (38) are arranged in a gas-tight manner.

## Revendications

1. Cuve (1) pour la mise en recirculation de matières organiques (2), par exemple de lisier ou par exemple pour le production de biogaz, comprenant une unité d'entraînement (4) avec un rotor (10) et un anneau/tuyau de circulation (7) pour la mise en recirculation des matières organiques (2) dans un système de conduite annulaire (5) de la cuve (1) et à travers une ouverture (11) dont la position verticale dans la cuve est modifiable dans une cloison (9) remplissant une section (8) du système de conduite annulaire (5), laquelle cloison (9) présente une percée (19) qui s'étend sur au moins une partie de la hauteur de la cloison (9) et il est prévu un rideau de cloison supérieur (12) et un rideau de cloison inférieur (13) par rapport à la profondeur de la cuve (1), qui couvrent une partie supérieure et une partie inférieure de la percée (19) et forment entre eux l'ouverture (11), les rideaux de cloison (12, 13) étant réalisés comme des éléments flexibles dont la longueur est variable et qui sont guidés dans des éléments de rail verticaux (16a, 16b), **caractérisée en ce qu'**une partie d'extrémité inférieure (31) du rideau de cloison inférieur (13) située à l'opposé de l'ouverture (11) peut être fixée en position dans une position de service au moyen d'un dispositif de maintien (32), de préférence dans une partie d'extrémité inférieure (21) de la percée (19) ou dans une partie d'extrémité inférieure de la cloison, le dispositif de maintien (32) pouvant être actionné à partir d'une zone située au-dessus de l'ouverture (11), de préférence de l'extérieur de la cuve (1), et le maintien en position de la partie d'extrémité inférieure (31) du rideau de cloison inférieur (13) pouvant ainsi être levé.

2. Cuve (1) pour la mise en recirculation de matières organiques (2) selon la revendication 1, **caractérisée en ce que** le dispositif de maintien (32) comprend deux éléments de barre (32a, 32b) allant de la partie d'extrémité supérieure (20) à la partie d'extrémité inférieure (21) de la percée (19) de la cloison (9) ou de la cloison (9) et disposées dans des parties d'extrémité latérales (24, 25) de la percée (19), dont les parties d'extrémité inférieures (32a', 32b') sont articulées sur la partie d'extrémité inférieure (31) du rideau de cloison inférieur (13).

3. Cuve (1) pour la mise en recirculation de matières organiques (2) selon la revendication 2, **caractérisée en ce que** des parties d'extrémité supérieures (32a" et 32b") des éléments de barre (32a, 32b) peuvent être fixées dans la partie d'extrémité supérieure (20) de la percée (19) ou de la cloison (9), la fixation des parties d'extrémité supérieures (32a", 32b") des éléments de barre (32a, 32b) fixant en position la partie d'extrémité inférieure (31) du rideau de cloison inférieur (13) dans sa position de service.

4. Cuve (1) pour la mise en recirculation de matières organiques (2) selon la revendication 2 ou 3, **caractérisée en ce que** les rideaux de cloison (12, 13) sont structurés en plusieurs segments repliables ou télescopiques par des entretoises (18) horizontales guidées dans les éléments dé rail (16a, 16b), l'articulation des parties d'extrémité inférieures (32a', 32b') du dispositif de maintien (32) sur la partie d'extrémité inférieure du rideau de cloison inférieur (13) s'effectuant par une entretoise (18) dans cette partie d'extrémité inférieure (31).

5. Cuve (1) pour la mise en recirculation de matières organiques (2) selon l'une des revendications 1 à 4, **caractérisée en ce que** le couvercle de cuve (33) de la cuve (1) présente au-dessus de la cloison (9) ou de l'unité d'entraînement (4) une trappe de visite (34) avec un passage (38) menant à l'intérieur de la cuve (1) pour un câble (39), pouvant être accroché directement ou indirectement à l'unité d'entraînement (4), d'un treuil à câble (35) disposé sur un portique de levage (36) passant au-dessus de la trappe de visite.

6. Cuve (1) pour la mise en recirculation de matières organiques (2) selon la revendication 5, **caractérisée en ce que** la trappe de visite (34) aussi bien que le passage (38) sont réalisés de façon étanche aux gaz.
